Europäisches Patentamt

European Patent Office

Office européen des brevets

(11) Veröffentlichungsnummer: **0 305 337 B1**

(12) **EUROPÄISCHE PATENTSCHRIFT**

(45) Veröffentlichungstag der Patentschrift: **07.10.92**

(51) Int. Cl.⁵: **G01N 33/546**, G01N 33/555, G01N 33/80

(21) Anmeldenummer: **88810576.4**

(22) Anmeldetag: **23.08.88**

Die Akte enthält technische Angaben, die nach dem Eingang der Anmeldung eingereicht wurden und die nicht in dieser Patentschrift enthalten sind.

(54) **Verfahren zum Nachweis von Antigenen und/oder Antikörpern sowie Testausrüstung zur Durchführung des Verfahrens.**

(30) Priorität: **24.08.87 CH 3240/87**

(43) Veröffentlichungstag der Anmeldung:
**01.03.89 Patentblatt 89/09**

(45) Bekanntmachung des Hinweises auf die Patenterteilung:
**07.10.92 Patentblatt 92/41**

(84) Benannte Vertragsstaaten:
**AT BE CH DE ES FR GB GR IT LI LU NL SE**

(56) Entgegenhaltungen:
EP-A- 0 039 195    EP-A- 0 194 156
EP-A- 0 194 212    EP-A- 0 224 439
DE-B- 1 005 759    FR-A- 2 236 181
FR-A- 2 554 240    GB-A- 2 017 910
US-A- 4 108 972

CHEMICAL ABSTRACTS, Band 94, Nr. 18, 04 Mai 1981, Columbus, OH (US); P.A.J.YAPA et al., Seite 59, Nr. 140920r

MICROBIOLOGY, Auflage 3, Harper Intl. Ed., 1980; DAVIS/DULBECCO et al., Seiten 324-327

PSCHYREMBEL, Klinisches Wörterbuch, Auflage 254, de Gruyter, 1982; Seite 61

(73) Patentinhaber: **STIFTUNG FÜR DIAGNOSTISCHE FORSCHUNG**
**Freiburgstrasse 65**
**CH-3280 Murten/FR(CH)**

(72) Erfinder: **LaPierre, Ives**
**36, Rue Ferrer**
**F-69600 Oullins(FR)**
Erfinder: **Josef, Dieter**
**Rte Henri-Dunant 17**
**CH-1700 Fribourg(CH)**
Erfinder: **Adam, Jean-R.**
**rue de l'Hôpital 45**
**CH-3280 Meyriez(CH)**
Erfinder: **Greber-Widmer, Susanne**
**Thalmatt 117**
**CH-3037 Herrenschwanden(CH)**

(74) Vertreter: **Fischer, Franz Josef et al**
**c/o Bovard AG Optingenstrasse 16**
**CH-3000 Bern 25(CH)**

EP 0 305 337 B1

EP 0 305 337 B1

**Beschreibung**

Die vorliegende Erfindung betrifft ein Verfahren zum Nachweis von Antigenen bzw. Antikörpern unter Verwendung von trägergebundenen Antikörpern sowie von inerten Partikeln, wie Polyacrylamidgelpartikel.

Trägergebundene Antigene bzw. Antikörper werden routinemässig für eine Vielzahl von analytischen Bestimmungen eingesetzt. Dabei werden generell zwei Prinzipien unterschieden:

a) das Antigen oder der Antikörper werden an einen festen Träger, wie Glas- oder Kunststoffröhrchen, Glas- oder Kunststoffkügelchen, Glas- oder Kunststoffplatten, Papier usw. gebunden und die Flüssigkeit mit dem zu bestimmenden Antikörper oder Antigen wird zugegeben. Nach einer gewissen Reaktionszeit sind diese, sofern sie mit dem gebundenen Antigen bzw. Antikörper reagieren, an den Träger gebunden und können mindestens durch eine weitere Reaktion messbar gemacht werden. In der Regel geschieht dies durch eine Markierung des Antikörpers oder Antigens mittels readioaktiven, fluoreszierenden oder enzymatisch aktiven Markiersubstanzen. Diese Methode hat den Nachteil, dass sie störanfällig ist. Da nach jeder Reaktion ein Waschen erforderlich ist, kann ungenügendes Waschen oder eine ungenügende Entfernung der Waschlösung die Resultate verfälschen. Dabei ist ebenfalls eine Aufspaltung des Antikörper-Komplexes oder eine unspezifische Adsorption nicht ausgeschlossen. Dieses Verfahren ist im übrigen für natürlich, trägergebundene Antigene auf Erythrozyten, Leukozyten, Thrombozyten und anderen natürlichen Zellen wegen deren Grösse schlecht anwendbar.

b) Eine weitere prinzipielle Methode besteht darin, dass das Antigen bzw. der Antikörper an kleine Partikel, wie Latex oder Erythrozyten gebunden wird. Nach einer gewissen Reaktionszeit mit dem zu bestimmenden Antikörper bzw. Antigen erfolgt die Beurteilung aufgrund des Agglutinationsmusters. Im Gegensatz zu der unter a) beschriebenen Methode, können nach diesem Verfahren Antigene bzw. Antikörper auf Erythrozyten, Leukozyten oder Thrombozyten bestimmt werden. Die Nachteile dieser Methode bestehen darin, dass insbesondere bei schwachen Konzentrationen nur schwer zwischen agglutinierten und nicht-agglutinierten (freien) Partikelchen unterschieden werden kann. Im weiteren können freie Partikelchen leicht am Reaktionsgefäss adsorbiert oder das Agglutinationsmuster kann leicht mechanisch zerstört werden. Eine weitere Fehlerquelle liegt in dem möglichen Zusammenkleben freier Partikel unter Vortäuschung einer Agglutination. Alle diese Vorgänge können zu einer falschen Beurteilung der Reaktion führen.

Aus der EP-A-0 039 195 ist ein Antikörper-Nachweisverfahren bekannt, worin Erythrozyten in negatiy geladener Form in einer isotonischen Lösung niederer Ionenstärke verwendet werden. Dabei wird als Agglutinationshilfsmittel die Lösung eines Polymers zugesetzt. Die Agglutination wird visuell vorzugsweise mittels eines Mikroskops beobachtet. Dabei kann das Agglutinat durch Zugabe einer Lösung von Citrat und Zucker wieder dissoziiert werden.

Ferner ist aus der EP-A-0 194 212 bekannt, dass beim Antigentest Erythrozyten, die in einer separaten Reaktion mit Antikörpern beladen werden, sich mit einem zweiten Antikörper in einem Gel von freien Erythrozyten durch Zentrifugation separieren lassen. Ueberraschenderweise wurde gefunden, dass ein zweiter Antikörper nicht notwendig ist, wenn die Grösse der Gelpartikel zwischen der Grösse der Erythrozyten und der Erythrozytenaggregate liegt und der erste Antiköper dem Gel zugemischt wird. Die Aggregate bilden sich hierbei beim Durchgang durch das Gel und werden durch die im Vergleich kleineren Gelpartikel nach oben gedrückt. Bei schwachen Antigenen oder Antikörpern gelingt der Test nur, wenn ihre Reaktionsfähigkeit verstärkt wird, sei es durch Freilegung verdeckter Antigene durch Enzyme, Verminderung der Ionenstärke im Puffer oder der negativen Ladung auf den Erythrozyten durch Kationen.

Bei äusserst schwachen Antigenen oder Antikörpern ist es ausserdem erforderlich, dass die Schwerkraft genau auf den Boden aller Reaktionsgefässe wirkt, damit eine Entscheidung positiv oder negativ erfolgen kann.

Bei den bisher üblichen Zentrifugen ist dies nicht der Fall, diese können jedoch durch Aenderung der Halterung leicht umgerüstet werden.

Aufgabe der vorliegenden Erfindung ist es, ein Nachweisverfahren für Antikörper oder Antigene zur Verfügung zu stellen, welches einfacher ist, als die bisher bekannten Verfahren, frei von deren Nachteilen ist und eine einfache und zuverlässige Ablesung erlaubt.

Es wurde gefunden, dass die obige Aufgabe durch die in der Kennzeichnung des Patentanspruchs 1 definierten Merkmale gelöst werden kann.

Gegenstand der vorliegenden Erfindung ist demzufolge das im Patentanspruch 1 definierte Verfahren und die in den Ansprüchen 16 bis 22 definierte Ausrüstung zur Durchführung des Verfahrens.

Die chemische Zusammensetzung der inerten Partikel, welche im Verfahren verwendet werden, ist nicht kritisch. Mit dem Merkmal inert kommt zur Geltung, dass die Partikel keine unspezifischen Reaktionen mit den Antigenen bzw. Antikörpern eingehen dürfen. Vorzugsweise werden solche inerte poröse Partikel

2

verwendet, wie sie im Handel für die Flüssig- oder Gaschromatographie (z.B. von Merck, LKB, Pharmacia und Biorad) erhältlich sind. Beispiele sind "Sephadex®", "Sepharose®", "Sephacryl®" oder "Bio-Gel®". Es handelt sich dabei um Produkte auf Basis von vernetzten Polymeren wie Agarose, Polyacrylamid, Polydextran oder Syrol-Divinylbenzolpolymere. Ebenfalls in Frage kommt poröses Glas oder Silikagel. Die Korngrössen betragen vorzugsweise 10 bis 200 $\mu$m. Der Fachmann kann durch einfach Vorversuche bestimmen, ob die Partikel für eine bestimmte Bestimmungsmethode brauchbar sind. Wie bei den obenerwähnten inerten Partikeln ist auch die Art des Trägers für das Antigen bzw. den Antikörpern nicht kritisch. Für visuelle bzw. optisch-automatische Messverfahren sollte er von Natur aus farbig sein (z.B. Erythrozyten). Die Träger können aber auch durch einen Farbstoff markiert sein (z.B. Latex, polymerisierte Agarose). Es kommen natürlich auch andere Markiersysteme in Frage, wie die Radio-, Fluoreszens- oder Enzymmarkierung. Diese erfordern selbstverständlich eine entsprechende Messmethode. Im übrigen sind die Träger vorzugsweise ebenfalls in Partikelform, wobei die Antigene bzw. Antikörper an deren Oberflächen gebunden sind.

Vorzugsweise sind die Antigene bzw. Antikörper an diese Partikel durch chemische Bindung gebunden, wobei die Art der Bindung nicht kritisch ist. Gewisse Antigene, wie diejenigen von Erythrozyten und Thrombozyten liegen bereits an diese Träger gebunden vor. Leukozyten und Thrombozyten können gegebenenfalls mit bekannten Methoden eingefärbt werden.

Im Fall der Leukozyten oder Thrombozyten können alternativ die inerten Partikel eingefärbt werden. In diesem Fall sind die Ablesezone weisslich und die inerten Partikel gefärbt.

Nach dem erfindungsgemässen Verfahren ist die Bestimmung der freien Antigene bzw. Antikörper möglich, wobei bestimmte trägergebundene Antikörper bzw. Antigene vorgegeben werden müssen. Umgekehrt können auch die trägergebundenen Antigene bzw. Antikörper bestimmt werden, Wobei die freien Antigene bzw. Antiköper vorgelegt werden müssen. Die zur Durchführung des Verfahrens verwendeten Reaktionsgefässe sind in der Regel Röhrchen oder Mikrotiterplatten aus Glas oder Kunststoff. Das Material ist nicht kritisch.

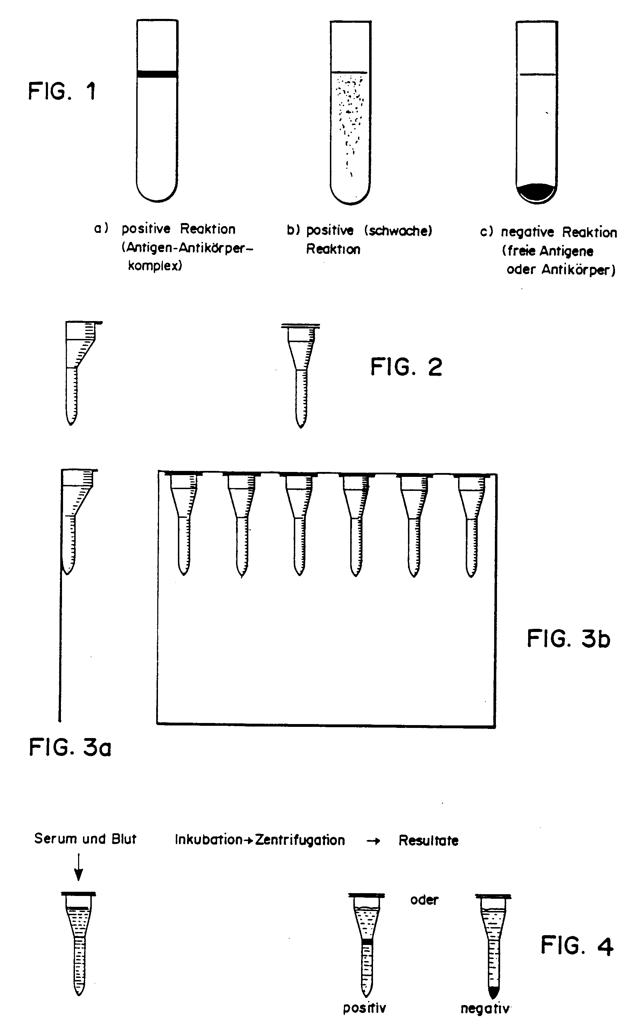

Für die Durchführung des erfindungsgemässen Verfahrens werden kleine Röhrchen oder Mikroreaktionsgefässe gemäss Fig. 2 verwendet. Diese können, wie in Fig. 3 angegeben, auf einer Karte in beliebiger Anzahl nebeneinander angeordnet sein. Es kann ein zusätzliches Gefäss vorhanden sein, beispielsweise für die Herstellung einer Probeverdünnung oder Probesuspension. Die Gefässe bzw. Karten können aus Kunststoff, wie PVC/PVDC, PET oder Polystyrol hergestellt sein. Beispielsweise können die Gefässe nach dem Blister-Verfahren, einem Schweiss-Verfahren oder durch Verleimung hergestellt sein. Die Gefässe können die inerten Partikel oder industriell vorbereitete Reaktionslösungen enthalten. Da im erfindungsgemässen Verfahren vorzugsweise zentrifugiert wird, müssen für die Röhrchen bzw. die Karten mit den Behältern spezielle passende Zentrifugenköpfe verwendet werden, die so konstruiert sind, dass die Schwerkraft genau senkrecht auf alle Böden der Reaktionsgefässe einwirkt. Ein vorteilhafter Zentrifugenkopf ermöglicht die gleichzeitige Zentrifugation von mindestens 12 Karten mit beispielsweise 6 Reaktionsgefässen. In diesem Fall können parallel 72 Tests durchgeführt werden.

Obwohl die Sedimentation durch Stehenlassen und Ausnutzen der Gravitationskräfte verursacht werden kann, wird vorteilhafterweise das Zentrifugenverfahren verwendet, da schon nach kurzer Zeit die gewünschte Sedimentation bewirkt werden kann. Die optimalen Bedingungen (Zentrifugationszeit und g-Zahl) müssen für jedes Analysesystem ermittelt werden, da das spezifische Gewicht, die Grösse, die Form, die Verformbarkeit und Stabilität des trägergebundenen Antigen-Antikörper-Komplexes, der nicht komplexierten, trägergebundenen Antikörper bzw. Antigene und der inerten Partikel einen schwer zu berechnenden Einfluss besitzen.

Anhand der Figuren werden gewisse Aspekte der Erfindung näher erläutert. Es zeigen:

**Fig. 1**      die bei positiver, schwach positiver und negativer Reaktion auftretenden Muster,

**Fig. 2**      eine Vorder- und eine Seitenansicht eines typischen Mikroröhrchens, wie es geeignet ist für die Aufbringung auf eine Karte,

**Fig. 3a und 3b**      eine Seiten- und Vorderansicht einer Karte mit 6 Röhrchen gemäss Fig. 2,

**Fig. 4**      in schematischer Weise die Testdurchführung und

**Fig. 5, 6 + 7**      Testkarten für typische klinische Tests, welche routinemässig durchgeführt werden.

Theoretisch kann das erfindungsgemässe Verfahren verwendet werden, um eine Testflüssigkeit nach einem Antigen bzw. Antikörper zu testen, welches spezifisch zu einem bekannten Antigen oder Antikörper ist. Entweder ist das bekannte Antigen bzw. Antikörper oder der unbekannte Antikörper bzw. Antigen an einen Träger, z.B. an einen Erythrozyten gebunden. Das Nachweisverfahren beruht auf der Erkenntnis, dass trägergebundene Antigene bzw. Antikörper und trägergebundene Antigen-Antikörper-Komplexe andere Zentrifugationseigenschaften aufweisen. Wird ein Antigen-Antikörper-Komplex, welcher an einem Träger ist, zusammen mit einem suspendierten, inerten Trägermaterial zentrifugiert, so liegt der trägergebundene Komplex auf den inerten Partikel. Hat keine Reaktion stattgefunden, befindet sich im Röhrchen neben dem inerten, suspendierten Material nur ein freies, trägergebundenes Antigen bzw. Antikörper. Nach der Zentrifugation befindet sich dieses unter der Schicht der inerten Partikel. Auf diese Weise kann deutlich visuell die positive oder negative Antikörper-Reaktion abgelesen werden. Es ist auch möglich, diese Reaktion zu automatisieren. Es können auch schwach positive Reaktionen auftreten, in diesem Fall befindet sich der trägergebundene Antigen-Antikörper-Komplex innerhalb der Schicht der inerten Partikel. Ein solches Muster ist in Fig. 1a dargestellt. Ist die Reaktion nur schwach positiv, d.h. dass nur wenige Antigen-Antikörper-Komplexe gebildet werden, so kann im oberen Teil der inerten Partikel des Zentrifugenglases der Antikörper nachgewiesen werden (Fig. 1b). Ist im Gegensatz dazu kein Antigen-Antikörper-Komplex vorhanden, sondern nur ein trägergebundenes, freies Antigen, bzw. ein trägergebundener, freier Antikörper, so lagert sich dieser nach der Zentrifugation unterhalb der inerten Partikel an. Dies ist in Fig. 1c dargestellt.

Die Fig. 1a bis 1c dienen lediglich zur Erläuterung der auftretenden Muster, wobei jedoch die Form des dargestellten Reaktionsgefässes nicht derjenigen entspricht, wie sie erfindungsgemäss zum Einsatz gelangt. Erfindungsgemäss wird ein solches Gefäss, wie es in Fig. 2 dargestellt ist, eingesetzt. Links befindet sich der Seitenriss und rechts eine Vorderaufsicht. Das Röhrchen besitzt einen Deckel, womit ermöglicht wird, dass gewisse industriell hergestellte Reagenzien bereits mit dem Teströhrchen geliefert werden können. Das Teströhrchen ist geeignet zur Aufbringung auf eine Karte. Eine solche Karte kann mehrere Röhrchen aufweisen. In Fig. 3 ist ein solches Beispiel dargestellt. Hier sind 6 Teströhrchen vorhanden. Fig. 3a zeigt eine Seitenansicht der Testkarte und Fig. 3b die Vorderansicht. Durch die Anordnung ist ein direkter Vergleich der parallelen Versuche möglich.

Die Testkarte kann auf verschiedene Arten hergestellt sein. Beispielsweise können Röhrchen auf eine Karte geklebt sein oder aber die Röhrchen können nach Art einer Blister-Packung in die Karte integriert ausgebildet sein. Dabei kann eine Mischung aus inerten Partikeln und Antikörpern oder Antigenen industriell luftdicht in vorbestimmter Menge in diese Röhrchen eingeschlossen sein, wobei die Röhrchen durch eine aufgeschweisste Folie verschlossen sein können. Auf diese Weise hergestellte Testausrüstungen ermöglichen eine einfachste Handhabung und können in einem automatischen Analysierverfahren verwendet werden. Dabei wird die Proben-Pipettierung, die Identifikation, das automatische Ablesen, die Auswertung, der Ausdruck usw. mittels elektrischer Datenverarbeitung gesteuert. Ein weiterer Vorteil liegt darin, das mit sehr kleinen Probemengen gearbeitet werden kann. Mit 10 bis 50 $\mu$l Blut lassen sich z.B. alle klinisch relevanten Antigene mit kleinen Reagenzienmengen nachweisen. Mikroansätze sind besonders wichtig bei chemisch-synthetisch nicht herstellbaren Substanzen, die nur in begrenzten Mengen zur Verfügung stehen, z.B. können mit 1 ml des Rhesus-Antikörpers-C mit einem konventionellen Verfahren 20 Antigen-Bestimmungen durchgeführt werden, während mit der beschriebenen Menge 1000 Bestimmungen möglich sind. Bei entsprechender Vorbereitung des Testsystems ist die Ausführung der Bestimmungen so einfach und die Resultate sind so eindeutig ablesbar, dass der Test auch von medizinischen Hilfspersonen durchgeführt werden kann.

Da keine Spezial-Laboratorien erforderlich sind, kann die Zeitspanne zwischen Diagnose und Therapiebeginn wesentlich verkürzt werden.

Ein weiterer Aspekt der vorliegenden Erfindung besteht in der Testausrüstung zur Durchführung des erfindungsgemässen Verfahrens, wie sie im Patentanspruch 16 definiert ist.

Die Erfindung wird durch die nachstehenden Beispiele näher erläutert.

## Beispiele

## Blutgruppen-Antigen (ABO-System)

### 1. Beispiel: A-Antigen

#### a) Vorbereitung der Suspension aus inerten Partikeln

5 ml Sephacryl, S200 Gel (Pharmacia) werden zweimal mit physiologischer Kochsalzlösung gewaschen. Nach Zentrifugation (5 Min., 1250 g) wird der Ueberstand verworfen und das Sediment mit isotonischem Imidazolpuffer (0,014 mol/l Imidazol 0,85 % NaCl) pH 7,6 auf 4,5 ml aufgefüllt.

#### b) Antikörperzugabe

Zu 4,5 ml obiger Suspension werden 0,5 ml Anti-A zugegeben. Die Suspension wird gut gemischt und ist in dieser Form gebrauchsfertig.

c) Vorbereitung der Reaktionsgefässe

In Mikroröhrchen aus Polyethylen (ET-29MM Milian SA) werden 100 $\mu$l obiger Antikörper-Suspension gegeben. Die inerten Partikel sedimentieren innerhalb weniger Minuten auf den Boden des Röhrchens.

d) Testausführung

20 $\mu$l einer ca. 4 % Erythrozytensuspension der unbekannten Blutprobe in isotonischem Imidazolpuffer pH 7,0 (1 Teil Blut und 9 Teile Puffer) werden in das mit Antikörper/Suspension gefüllte Reaktionsgefäss gegeben und 10 Min. bei ca. 100 g (Zentrifuge Heraeus Digifuge GL 122 089, 800 U/Min.) zentrifugiert.

e) Auswertung

Gehört das untersuchte Blut zur Blutprobe A ($A_1$ oder $A_2$), so liegt der Antigen-Antikörper-Komplex nach der Zentrifugation auf den inerten Partikeln (Fig. 1a). Gehört das Blut zu den seltenen A-Untergruppen $A_3$ oder $A_x$, so verteilt sich der Komplex zwischen den inerten Partikeln (Fig. 1b). Gehört das Blut zu den Gruppen B oder O, so kann keine Agglutination erfolgen, die Erythrozyten sammeln sich nach der Zentrifugation auf dem Boden des Röhrchens unter den inerten Partikeln (Tabelle 1).

Tabelle 1

| Blutgruppe | $A_1/A_2/A_1 B/A_2 B$ | $A_3/A_3 B/Ax$ A int. | B/O |
|---|---|---|---|
| Test | positiv (Fig. 1a) | schwach positiv (Fig. 1b) | negativ (Fig. 1c) |

Völlig analog zum Beispiel 1 wird das B-Antigen mit Anti-B anstelle des Anti-A bestimmt. Das H-Antigen lässt sich mit H-Lectin nachweisen. Eine Differenzierung zwischen $A_1$ und $A_2$ erfolgt mit $A_1$-Lectin. Das Beispiel 1 lässt eine Vielzahl Variationen der inerten Partikel, der verwendeten Puffer, der Reaktionsgefässe, der Zentrifugationszeit und g-Zahl zu. Die Herkunft der Antikörper, ob human, tierisch oder pflanzlich, ob polyklonal oder monoklonal, ist unwesentlich, vorausgesetzt die Variationen verändern nicht das Reaktionsbild (Fig. 1).

**II. Blutgruppen Rh-System (D, Du, C, E, c, e, Cw)**

2. Beispiel: E-Antigen

Die Vorbereitung der Reaktionsgefässe (Schritte a - c) ist wie im 1. Beispiel. Anstelle des Anti-A wird z.B. Anti-E verwendet.

d) Testausführung

Der Schritt d) in Beispiel 1 wird so geändert, dass anstelle des Imidazolpuffers eine Enzymlösung (DiaBrom, DiaMed AG) verwendet wird. Es ist bekannt, dass dadurch verdeckte Rhesusantigene freigelegt werden können. 50 $\mu$l Blut werden in 0,5 ml Enzymlösung suspendiert. Nach ca. 5 Min. werden 20 $\mu$l dieser Suspension in das nach Beispiel 1, Schritt a) - c), präparierte Reaktionsgefäss gegeben und 10 Min. bei 100 g zentrifugiert.

e) Auswertung

Enthält die Blutprobe das Antigen E, so liegt der Antigen-Antikörper-Komplex auf den inerten Partikeln (Tabelle 2). Ist das Antigen E nicht vorhanden, so sammeln sich die Erythrozyten unter den inerten Partikeln.

Tabelle 2

|  | E-Antigen vorhanden | E-Antigen abwesend |
|---|---|---|
| Test | positiv (Fig. 1a) | negativ (Fig. 1c) |

Völlig analog lassen sich die anderen Rhesusantigene mit den entsprechenden Antiseren bestimmen. Eine Differenzierung der klinisch wichtigen D-Variante Du kann durch Variation der Konzentration des Anti-D erfolgen.

**II. Antikörper**

3. Beispiel: Serumgegenprobe

Die Vorbereitung der Reaktionsgefässe (Schritte a) - c) ist wie im Beispiel 1, nur wird der Antikörper durch Imidazolpuffer ersetzt.

d) Testdurchführung

Da in diesem Beispiel Antikörper identifiziert werden sollen, werden Erythrozyten mit bekannten Antigenen verwendet, z.B. A-, B- und O-Testerythrozyten. Diese werden in der bekannten LISS-Lösung suspendiert (50 $\mu$l Blut und 2,0 ml LISS). In drei identisch gefüllte Reaktionsgefässe werden 50 $\mu$l Serum oder Plasma der unbekannten Blutprobe gegeben.

In das 1. Röhrchen fügt man 100 $\mu$l der A-Zellen-Suspension, in das 2. Röhrchen 100 $\mu$l der B-Zellen-Suspension und in das 3. Röhrchen 100 $\mu$l der O-Zellen-Suspension in LISS. Wie in den Beispielen 1 und 2 wird 10 Min. bei 100 g zentrifugiert.

e) Auswertung

Enthält die unbekannte Probe Anti-A, so ist das 1. Röhrchen positiv, das 2. und 3. negativ (Tabelle 3).

Tabelle 3

|  | A-Zellen | B-Zellen | O-Zellen |
|---|---|---|---|
| Test | positiv (Fig. 1a) | negativ (Fig. 1c) | negativ (Fig. 1c) |

Enthält die Probe Anti-B, so ist das 2. Röhrchen positiv und das 1. und 2. negativ. Ist das Röhrchen 3 positiv und 1 und 2 positiv oder negativ, so enthält die Probe Antikörper, die nicht Anti-A oder Anti-B sind, sondern gegen andere Antigene, die auf den O-Zellen liegen, gerichtet sind. In diesem Falle sind weitere Abklärungen erforderlich.

4. Beispiel: Antikörper-Suchtest (Coombs-Test)

Die Vorbereitungen der Reaktionsgefässe sind wie in den Beispielen 1 - 3. Nur wird der Suspension aus inerten Partikeln Coombs-Serum (Diamed AG) zugesetzt. Das Coombs-Serum besteht bekanntermassen aus Anti-human-IgG und Anti-Komplement C3 (C3b + C3d), wie auch Anti-IgM und Anti-IgA und dient zur Erfassung und Identifizierung von Antikörpern im Patientenserum, die gegen Erythrozyten-Antigene gerichtet sind. Zweckmässigerweise wird erst ein Antikörper-Suchtest durchgeführt, bei dem O-Erythrozyten mit bekanntem Antigenmuster für klinisch relevante Antikörper verwendet werden. Bei positivem Ausfall des Suchtests erfolgt eine Identifikation mit einem Zellpanel.

Testdurchführung

In ein oder mehrere identische mit Coombs-Serum-Suspension gefüllte Reaktionsgefässe werden 50 $\mu$l Patientenserum oder Plasma in die Ausbuchtung oder den Trichter des Reaktionsgefässes gefüllt. Dazu werden 100 $\mu$l der 0-Zellen-Suspension (50 $\mu$l Blut in 2,0 ml LISS) gegeben. Die Mischung wird 10-20 Min.

6

bei 37°C, RT oder 2 - 8°C, je nach gesuchtem Antikörper, inkubiert und 10 Min. bei 100 g zentrifugiert. Die Ablesung erfolgt wie in den vorherigen Beispielen.

Enthält die Probe einen Antikörper gegen ein oder mehrere Erythrozyten-Antigene, so ergibt sich das positive Muster gemäss Fig. 1a. Bei schwachen Antikörpern ist das Muster wie in Fig. 1b. Analog zur obigen Testdurchführung kann der entdeckte Antikörper mit einem Zellpanel, das unterschiedliche Antigene enthält, identifiziert werden (z.B. Handelsprodukte Ortho Dade, DiaMed).

### IV. Blutgruppenbestimmung auf einer Karte

5. Beispiel: Blutgruppe A, $R_1R_2$ (CCDee) Kell negativ, Kontrollen i.O.

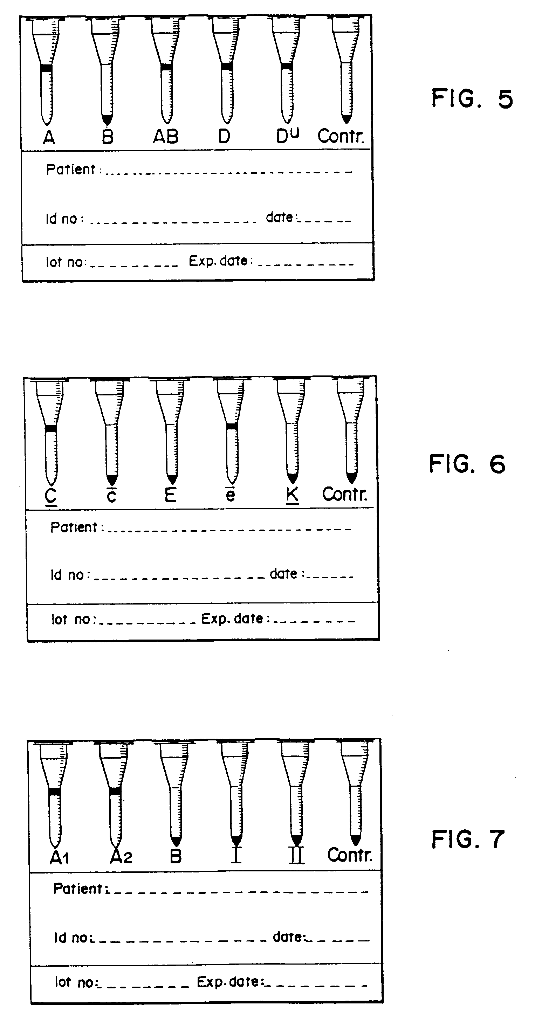

Die Bestimmungen können einzeln in Röhrchen, auf Mikrotiterplatten oder auf Karten ausgeführt werden. In diesem Beispiel wird eine Blutgruppen bestimmung auf Karten beschrieben, die für die Bestimmung der Blutgruppe eines Patienten konzipiert ist. Die Präparation der Reaktionsgefässe erfolgt wie vorher beschrieben. Die Karten sind in den Fig. 5 - 7 dargestellt.

### V. Bestimmung von freien Antigenen bzw. Antikörpern, die nicht zum Blutgruppen-System gehören

Die Reaktionen in den Beispielen 1 - 5, bei denen das Antigen von Natur aus an Erythrozyten gebunden ist, lassen sich auf freie Antigene bzw. Antikörper anwenden, indem die korrespondierenden Antikörper bzw. Antigene mit bekannten Methoden an fixierte Erythrozyten bzw. andere Partikel gebunden werden.

6. Beispiel: Rheumatest

a) Präparation der Erythrozyten

5 ml Ziegenblut in 0,011 mol Citratpuffer pH 6 werden dreimal mit physiologischer Kochsalzlösung gewaschen. Das Sediment wird mit 5 ml physiologischer Kochsalzlösung suspendiert, mit 0,5 ml 30 % Glutaraldehydlösung (Merck) versetzt und 24 Std. bei Raumtemperatur unter Rühren reagieren gelassen. Das Sediment wird dreimal mit physiologischer Kochsalzlösung gewaschen und mit 0,5 ml Kaninchen-IgG (10 mg/ml) versetzt und 24 Std. bei Raumtemperatur unter Rühren inkubiert. Nach dreimaligem Waschem mit physiologischer Kochsalzlösung wird eine ca. 40 % Suspension der beladenen Erythrozyten in Imidazolpuffer hergestellt.

Testdurchführung

Die Reaktionsgefässe werden wie in Beispiel 3 vorbereitet. Mit Imidazolpuffer wird eine ca. 4 %-ige Erythrozyten-Suspension hergestellt. In die Ausbuchtung des Reaktionsröhrchens werden wie in Beispiel 4 50 µl Patientenserum gefüllt. Dazu werden 20 µl der 4 %-igen Eryhtrozyten-Suspension gegeben. Nach ca. 10 Min. Inkubation bei Raumtemperatur wird wie üblich zentrifugiert. Enthält das Serum Rheumafaktoren, so zeigt sich das Reaktionsmuster wie in Fig. 1a oder b. Will man den Titer ermitteln, so wird der Test mit entsprechend verdünntem Serum (0,9 % NaCl) wiederholt.

Natürlich ist der beschriebene Test nicht auf die Rheumafaktoren beschränkt, z.B. liessen sich nach Kopplung von HBsAg-Antikörpern Hepatitis-Antigene nachweisen. Durch Kopplung von abgetöteten HIV-Viren oder deren synthetisch hergestellte Hüllproteine an die Erythrozyten lassen sich ebenfalls einfach HIV-Antikörper nachweisen.

Anhand dieser Beispiele ist unmittelbar einleuchtend, dass nach dem gleichen Verfahren auch andere Proteine, Viren oder Bakterien oder deren Antikörper leicht bestimmt werden können.

### Patentansprüche

1. Verfahren zum Nachweis von Antikörpern bzw. Antigenen durch optische Sichtbarmachung von trägergebundenen Komplexen von Antigenen mit Antikörpern in wässerigem Medium in einem Reaktionsgefäss, worin eine Lösung, welche Antikörper bzw. Antigene enthält, mit trägergebundenen Antigenen bzw. Antikörpern in Kontakt gebracht wird, wobei vor diesem Schritt eine Aufschlämmung oder Suspension von inerten Partikeln in das Reaktionsgefäss gegeben wird, und anschliessend die Mischung zur Sedimentation der Gravitation ausgesetzt wird, wobei bei der Bildung eines Antigen-Antikörper-Komplexes, dieser im stark positiven Fall auf dem Sediment der inerten Partikel liegt und im schwach positiven Fall innerhalb der inerten Partikel vorhanden ist und bei Abwesenheit eines Antigen-Antiköper-Komplexes, d.h. im negativen Fall unter den sedimentierten inerten Partikeln liegt, dadurch gekennzeichnet, dass das gesamte Verfahren in einem einzigen Mikroreaktionsgefäss gemäss den Fig. 2 oder 3 durchgeführt wird.

2. Verfahren nach Anspruch 1, dadurch gekennzeichnet, dass zum optischen Nachweis der Träger an welchen das Antigen oder der Antikörper gebunden ist, eine natürliche Färbung aufweist oder markiert ist.

3. Verfahren nach Anspruch 2, dadurch gekennzeichnet, dass der Träger farb-, radio-, fluoreszenz oder enzymmarkiert ist.

4. Verfahren nach Anspruch 2 oder 3, dadurch gekennzeichnet, dass der Träger aus Erythrozyten, Leukozyten oder Thrombozyten besteht.

5. Verfahren nach Anspruch 4, dadurch gekennzeichnet, dass der Träger zur Verstärkung der Reaktion mit proteolytischem Enzym vorbehandelt wird.

6. Verfahren nach Anspruch 5, dadurch gekennzeichnet, das das Enzym Papain, Ficin oder Bromelin ist.

7. Verfahren nach einem der Ansprüche 1 bis 3, dadurch gekennzeichnet, dass der Träger an welchen das Antigen oder der Antikörper gebunden ist, aus Latex oder polymerisierter Agarose besteht.

8. Verfahren nach einem der Ansprüche 1 bis 7, dadurch gekennzeichnet, dass die inerten Partikel poröse Polyacrylamidgel-Kügelchen, Kügelchen aus vernetztem Dextran oder Kieselgel-Kügelchen mit einer Korngrösse von 10 bis 200 $\mu$m sind

9. Verfahren nach einem der Ansprüche 1-8 dadurch gekennzeichnet, dass die Probemischung der Gravitation ausgesetzt wird, indem sie einer Zentrifugation unterworfen wird.

10. Verfahren nach Anspruch 9, dadurch gekennzeichnet, dass für die Zentrifugation ein Zentrifugenkopf verwendet wird, welcher bewirkt, dass die Schwerkraft genau auf den Boden des Reaktionsgefässes wirkt.

11. Verfahren nach einem der Ansprüche 1-8 dadurch gekennzeichnet, dass es in einem isotonischen Puffer bei einem pH-Wert von 4 bis 8 durchgeführt wird.

12. Verfahren nach Anspruch 11 dadurch gekennzeichnet, dass die Reaktion in einem Puffer niedriger Ionenstärke durchgeführt wird.

13. Verfahren nach einem der Ansprüche 1-12, dadurch gekennzeichnet, dass es zur Bestimmung der Blutgruppen dient.

14. Verfahren nach einem der Ansprüche 1-12 dadurch gekennzeichnet, dass die trägergebundenen Antikörper gegen Proteine, Viren, Bakterien oder Hüllproteine gerichtet sind.

15. Verfahren nach Anspruch 11, dadurch gekennzeichnet, dass die trägergebunden Antigene Bestandteile von Körperflüssigkeiten, z,B. Blut, Serum oder Plasmabestandteile sind.

16. Testausrüstung zum Nachweis von Antigenen oder Antikörpern nach dem Verfahren nach einem der Ansprüche 1-15, dadurch gekennzeichnet, dass sie zumindest ein Mikroreaktionsgefäss gemäss Fig. 2 oder 3, inerte Partikel und pro Reaktionsgefäss einen trägergebundenen Antiköper oder Antigen enthält.

17. Testausrüstung zum Nachweis von trägergebundenen Antigenen oder Antikörpern nach dem Verfahren nach einem der Ansprüche 1-15, dadurch gekennzeichnet, dass zumindest ein Mikroreaktionsgefäss gemäss Fig. 2 oder 3, inerte Partikel und pro Reaktionsgefäss einen Antiköper oder Antigen enthält.

**18.** Testausrüstung gemäss Anspruch 16 oder 17, zur gleichzeitigen Durchführung mehrerer Tests, dadurch gekennzeichnet, dass sie mehrere Mikroreaktionsgefässe gemäss Fig. 2 oder 3 umfasst, die übersichtlich gruppiert und miteinander verbunden sind.

**19.** Testausrüstung nach Anspruch 18, dadurch gekennzeichnet, dass die Mikroreaktionsgefässe parallel nebeneinander auf einer Karte angeordnet sind.

**20.** Testausrüstung nach Anspruch 19, dadurch gekennzeichnet, dass sie zusätzlich einen Zentrifugenkopf umfasst, welcher zur Aufnahme mehrerer Karten ausgebildet ist, wobei die eingesetzten Karten schwenkbar in Ebenen angeordnet sind, welche Ebenen als gemeinsames Element die Zentrifugendrehachse enthalten, und beim Betrieb der Zentrifuge die Karten so ausgerichtet werden, dass die Achsen der Mikroreaktionsgefässe in einen rechten Winkel zur Zentrifugendrehachse gebracht werden, damit die Zentrifugenkraft genau auf den Boden der Mikroreaktionsgefässe einwirkt.

**21.** Testausrüstung nach einem der Ansprüche 16-20, dadurch gekennzeichnet, dass die für den Test zu verwendenden Komponenten separat abgepackt sind, um im Zeitpunkt der Testdurchführung mit der Probe zusammen in das entsprechende Mikroreaktionsgefäss abgefüllt zu werden.

**22.** Testausrüstung nach einem der Ansprüche 16-20, dadurch gekennzeichnet, dass die Mikroreaktionsgefässe durch Deckel verschlossen sind und Komponenten zur Durchführung des Verfahrens enthalten.

**Claims**

**1.** A method for the detection of antibodies and/or antigens by optically making visible carrier-bound antigen complexes with antibodies in an aqueous medium in a reaction vessel, wherein a solution containing antibodies or antigens is brought into contact with carrier-bound antigens or antibodies, a slurry or a suspension of inert particles being added to the reaction vessel prior to this step, and the mixture subsequently being exposed to sedimentation of the gravitation, wherein if an antigen-antibody complex is formed, in a strongly positive reaction it lies on the sediment of the inert particles and in a weakly positive reaction it is present within the inert particles, and in the absence of an antigen-antibody complex, ie. in a negative reaction it lies beneath the sedimented inert particles, characterized in that the entire method is carried out in a single micro reaction vessel according to Fig. 2 or 3.

**2.** The method of claim 1, characterized in that for optical detection the carrier to which the antigen or the antibody is bound is naturally coloured or tagged.

**3.** The method of claim 2, characterized in that the carrier is tagged by colour, isotope, fluorescence or enzyme.

**4.** The method of claim 2 or 3, characterized in that the carrier consists of erythrocytes, leukocytes, or platelets.

**5.** The method of claim 4, characterized in that the carrier is pretreated with proteolytic enzyme to intensify the reaction.

**6.** The method of claim 5, characterized in that the enzyme is papain, ficin or bromelin.

**7.** The method of one of claims 1 to 3, characterized in that the carrier to which the antigen or antibody is bound, is made of latex or polymerized agarose.

**8.** The method of one of claims 1 to 7, characterized in that the inert particles are globules of porous polyacrylamide gel, of cross-linked dextran, or of silica gel with a particle size of 10 to 200 $\mu$m.

**9.** The method of one of claims 1-8, characterized in that the sample mixture is exposed to gravitation by subjecting it to centrifugation.

**10.** The method of claim 9, characterized in that a centrifuge head causing the force of gravity to take effect precisely on the bottom of the reaction vessel is used for the centrifugation.

**11.** The method of one of claims 1-8, characterized in that it is carried out in an isotonic buffer at a pH of 4 to 8.

**12.** The method of claim 11, characterized in that the reaction is carried out in a buffer of low ionic strength.

**13.** The method of one of claims 1-12, characterized in that it is used to type blood groups.

**14.** The method of one of claims 1-12, characterized in that the carrier-bound antibodies are directed against proteins, viruses, bacteria or coat proteins.

**15.** The method of claim 11, characterized in that the carrier-bound antigens are components of body fluids, eg. blood, serum or plasma components.

**16.** A test kit for detecting antigens or antibodies according to the method of one of claims 1-15, characterized in that it contains at least one micro reaction vessel according to Fig. 2 or 3, inert particles and a carrier-bound antibody or antigen per reaction vessel.

**17.** A test kit for detecting carrier-bound antigens or antibodies according to the method of one of claims 1-15, characterized in that it contains at least one micro reaction vessel according to Fig. 2 or 3, inert particles and one antibody or antigen per reaction vessel.

**18.** The test kit of claim 16 or 17 for simultaneously carrying out several tests, characterized in that it comprises several micro reaction vessels according to Fig. 2 or 3, said vessels being plainly grouped and interconnected.

**19.** The test kit of claim 18, characterized in that the micro reaction vessels are disposed parallel next to each other on a card.

**20.** The test kit of claim 19, characterized in that it also comprises a centrifuge head designed to accept several cards, the cards used being pivotably disposed in planes which contain the axis of rotation of the centrifuge as a common element, and when the centrifuge is operated, the cards are directed so that the axes of the micro reaction vessels are brought to a right angle relative to the pivot axis of the centrifuge so that the centrifugal force takes effect precisely on the bottom of the micro reaction vessels.

**21.** The test kit of one of claims 16-20, characterized in that the components to be used for the test are separately packaged for insertion in the appropriate micro reaction vessel at the time of testing together with the sample.

**22.** The test kit of one of claims 16-20, characterized in that the micro reaction vessels are closed by means of lids, and contain components for carrying out the method.

**Revendications**

**1.** Procédé de détection des anticorps, resp. des antigènes par la mise en évidence optique de complexes liés à un support d'antigènes avec des anticorps en milieu aqueux dans un récipient de réaction, dans lequel on met en contact une solution contenant des anticorps, resp. des antigènes, avec des antigènes, resp. des anticorps, liés à un support, une bouillie ou une suspension de particules inertes étant mise dans le récipient de réaction avant cette étape, ensuite de quoi on soumet le mélange à sédimenter à la gravitation, ce par quoi lorsqu'un complexe d'antigènes-anticorps se forme, dans une réaction très positive, il se trouve sur le sédiment des particules inertes, et en réaction faiblement positive, il est présent à l'intérieur des particules inertes, et en l'absence de formation d'un complexe antigènes-anticorps, c'est-à-dire dans une réaction négative, il se trouve sous les particules inertes sédimentées, caractérisé en ce qu'on effectue l'ensemble du procédé dans un seul micro-récipient de réaction selon la figure 2 ou la figure 3.

**2.** Procédé selon la revendication 1, caractérisé en ce que pour la détection optique le support, auquel l'antigène ou l'anticorps est lié, présente une couleur naturelle ou est marqué.

3.  Procédé selon la revendication 2, caractérisé en ce que le support est marqué par coloration, de manière radio-active, fluorescence ou enzymatique.

4.  Procédé selon la revendication 2 ou la revendication 3, caractérisé en ce que le support consiste en des érythrocytes, des leucocytes ou des thrombocytes.

5.  Procédé selon la revendication 4, caractérisé en ce que pour renforcer la réaction le support est prétraité avec une enzyme protéolytique.

6.  Procédé selon la revendication 5, caractérisé en ce que l'enzyme est la papaïne, ficine ou broméline

7.  Procédé selon l'une des revendications 1 à 3, caractérisé en ce que le support auquel l'antigène ou l'anticorps est lié se compose de latex ou d'agarose polymérisée.

8.  Procédé selon l'une des revendications, 1 à 7, caractérisé en ce que les particules inertes sont des granules de gel polyacrylamide poreux, des granules de dextran réticulé ou des granules de gel de silice ayant une grosseur de particule de 10 à 200 $\mu$m.

9.  Procédé selon l'une des revendications 1-8, caractérisé en ce qu'on expose le mélange de test à la gravitation en le soumettant à une centrifugation.

10. Procédé selon la revendication 9, caractérisé en ce que pour la centrifugation on utilise une tête centrifugeuse ayant pour effet que la force de gravité agit exactement sur le fond du récipient de réaction.

11. Procédé selon l'une des revendications 1-8, caractérisé en ce qu'on l'exécute dans un tampon isotonique à un pH de 4 à 8.

12. Procédé selon la revendication 11, caractérisé en ce qu'on conduit la réaction dans un tampon ayant une force ionique plus basse.

13. Procédé selon l'une des revendications 1-12, caractérisé en ce qu'il sert à déterminer les groupes sanguins.

14. Procédé selon l'une des revendications 1-12, caractérisé en ce que les anticorps liés au support sont dirigés contre les protéines, les virus, les bactéries ou les protéines-enveloppes.

15. Procédé selon la revendication 11, caractérisé en ce que les antigènes liés au support sont des composants des liquides du corps, par exemple des composants du sang, du sérum et du plasma.

16. Nécessaire pour test de détection d'antigènes ou d'anticorps selon le procédé selon l'une des revendications 1-15, caractérisé en ce qu'il contient au moins un micro-récipient de réaction selon la fig. 2 ou la fig. 3, des particules inertes et un anticorps ou un antigène lié au support par récipient de réaction.

17. Nécessaire pour test de détection d'antigènes ou d'anticorps selon le procédé selon l'une des revendications 1-15, caractérisé en ce qu'il contient au moins un micro-récipient de réaction selon la fig. 2 ou la fig. 3, des parties inertes et un anticorps ou un antigène par récipient de réaction.

18. Nécessaire pour test selon la revendication 16 ou la revendication 17 pour l'exécution simultanée de plusieurs tests, caractérisé en ce qu'il comprend plusieurs micro-récipients de réaction selon la fig. 2 ou la fig. 3 qui sont clairement groupés et reliés les uns aux autres

19. Nécessaire pour test selon la revendication 18, caractérisé en ce que les micro-récipients de réaction sont disposés en parallèle les uns à côtés des autres sur une carte.

**20.** Nécessaire pour test selon la revendication 19, caractérisé en ce qu'il comprend en plus une tête de centrifugation qui est configurée pour la prise en charge de plusieurs cartes, les cartes utilisées étant disposées de manière pivotante en couches, ces couches contenant l'axe de la centrifugeuse comme élément commun, et lorsque la centrifugeuse fonctionne les cartes s'orientent de telle manière que les axes des micro-récipients de réaction forment un angle droit par rapport à l'axe de la centrifugeuse pour que la force centrifuge agisse exactement sur le fond des micro-récipients de réaction.

**21.** Nécessaire pour test selon l'une des revendications 16 à 20, caractérisé en ce que les composants à utiliser pour le test sont emballés séparément de façon que, au moment de l'exécution du test, ils soient chargés avec l'échantillon dans le micro-récipient approprié.

**22.** Nécessaire pour test selon l'une des revendications 16 à 20, caractérisé en ce que les micro-récipients de réaction sont fermés par des couvercles et contiennent les composants pour l'exécution du procédé.

FIG. 1

a) positive Reaktion
(Antigen-Antikörper-
komplex)

b) positive (schwache)
Reaktion

c) negative Reaktion
(freie Antigene
oder Antikörper)

FIG. 2

FIG. 3b

FIG. 3a

Serum und Blut     Inkubation→Zentrifugation     →     Resultate

oder

FIG. 4

positiv                negativ

FIG. 5

FIG. 6

FIG. 7